(19) Europäisches Patentamt
European Patent Office
Office européen des brevets

(11) Veröffentlichungsnummer : **0 316 374 B1**

# (12) EUROPÄISCHE PATENTSCHRIFT

(45) Veröffentlichungstag der Patentschrift : **31.07.91 Patentblatt 91/31**

(51) Int. Cl.$^5$ : **C07C 69/16**, C07C 69/28, C07C 31/13, C12P 1/00, C07D 311/94

(21) Anmeldenummer : **88903165.4**

(22) Anmeldetag : **02.05.88**

(86) Internationale Anmeldenummer : **PCT/CH88/00083**

(87) Internationale Veröffentlichungsnummer : **WO 88/08838 17.11.88 Gazette 88/25**

## (54) OPTISCH AKTIVE VERBINDUNGEN.

(30) Priorität : **06.05.87 CH 1730/87**

(43) Veröffentlichungstag der Anmeldung : **24.05.89 Patentblatt 89/21**

(45) Bekanntmachung des Hinweises auf die Patenterteilung : **31.07.91 Patentblatt 91/31**

(84) Benannte Vertragsstaaten : **AT BE CH DE FR GB IT LI NL**

(56) Entgegenhaltungen : **Keine Entgegenhaltungen**

(73) Patentinhaber : **F. HOFFMANN-LA ROCHE AG
Postfach 3255
CH-4002 Basel (CH)**

(72) Erfinder : **SCHMID, Rudolf
Im Weissgrien 24
CH-4142 Münchenstein (CH)**
Erfinder : **WIRZ, Beat
Wiedenweg 4
CH-4153 Reinach (CH)**

(74) Vertreter : **Urech, Peter, Dr. et al
Grenzacherstrasse 124 Postfach 3255
CH-4002 Basel (CH)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft neue, optisch aktive Verbindungen, nämlich optisch aktive Derivate von 2-Methyl-1,3-propandiol, und zwar die Verbindungen der Formel

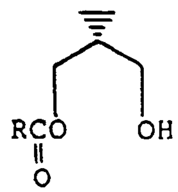

I

worin R $C_{1-10}$-Alkyl, insbesondere $C_{1-5}$-Alkyl, darstellt.

Der Methyl-Substituent der oben angegebenen optisch aktiven Strukturformel liegt, wie durch das Zeichen ((R)-Konfiguration) gekennzeichnet, hinter der Molekülebene.

Die Erfindung betrifft auch ein Verfahren zur Herstellung der Verbindungen I. Dieses Verfahren ist dadurch gekennzeichnet, dass man einen symmetrischen Diester der Formel

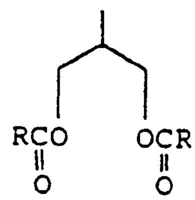

II

worin R obige Bedeutung besitzt,
mit einer Lipase, vorzugsweise einer mikrobiellen Lipase des Genus Pseudomonas, behandelt.

Die Verbindungen I sind wertvolle Zwischenprodukte, insbesondere für die Vitamin E-Chemie : Sie eröffnen insbesondere vorteilhafte Synthesewege für die Seitenkette von optisch aktivem Vitamin E.

R ist insbesondere ein n-Alkylrest, also z.B. Methyl, Aethyl, Propyl, Butyl, Amyl, Hexyl, Decyl, etc.

Bevorzugt sind die n-$C_{1-5}$-Radikale, speziell die n-$C_{2-4}$-Radikale.

Aber auch verzweigte Alkylreste, z.B. endständig verzweigte Alkylreste, wie Isobutyl oder Isoamyl kommen in Frage.

Der Alkylrest kann schliesslich auch substituiert sein, z.B. einen ω-Haloalkylrest darstellen ; Beispiele dafür sind Chlormethyl, 2-Chloräthyl, 3-Chlorpropyl, 4-Chlorbutyl.

Die asymmetrische Hydrolyse der prochiralen Precursoren, der Diester II, wird erfindungsgemäss unter Einwirkung einer Lipase, vorzugsweise einer mikrobiellen Lipase des Genus Pseudomonas, durchgeführt.

Geeignete Enzympräparate sind Lipase P30 von Amano Pharmaceuticals Co., Ltd. 2-7, 1-chome, Nishiki, Naka-ku, Nagoya 460, Japan, eine mikrobielle Lipase aus Pseudomonas fluorescens.

Weitere Produkte sind Lipoprotein Lipase aus einer Pseudomonas Species der Firma Sigma Chemie GmbH, Am Bahnsteig 7, D-8028 Taufkirchen, Deutschland ; Lipase aus einer Pseudomonas sp. von Boehringer Mannheim (Schweiz) AG, Industriestrasse, CH-6343 Rotkreuz, oder auch Lipasen aus Rhizopus sp., z.B. Rhizopus arrhizus von Sigma oder Lipase aus Mucor sp., z.B. Lipase M-AP-10 aus Mucor javanicus von Amano.

Das erstgenannte Produkt ist bevorzugt.

Die zweckmässigen Parameter der enzymatischen Hydrolyse sind die folgenden :

Medium : organisch/wässrige Emulsion ; organische Phase : das mit Wasser nicht mischbare Diestersubstrat II, bevorzugt ohne Lösungsmittelzusatz, gegebenenfalls aber zuzüglich mit Wasser nicht mischbarer, unpolarer oder polarer Lösungsmittel, wie n-Hexan, i-Octan, Aether, etc.
wässrige Phase : $H_2O$
Puffer, z.B. Natriumphosphat, Natriumcitrat, etc.

c = 1 mM – 1 M, insbesondere 3 mM – 0,1 M

Additive (fakultativ), wie
— salting-in salts ("Einsalz"-salze) :

z.B. Lithiumrhodanid
Guanidiniumrhodanid
Guanidiniumchlorid

c = 0,05 – 2 M, bevorzugt 0,05 – 0,5 M

— mono- oder polyhydrische Alkohole
z.B. Aethanol
Propan-1,3-diol
Glycerin
Mannitol

c = 0,05 – 4 M, bevorzugt 0,05 – 0,5 M

— Natriumcitrat
Natriumtartrat

c = 0,05 – 2 M, bevorzugt 0,05 – 0,5 M.

Emulgatoren (fakultativ) : handelsübliche Produkte der Triton-, Tween-, Span-, Brij-Reihen, Polyvinylalkohole
Substratkonzentration : 0,5-50% (G/V), bevorzugt 0,5-10% (G/V)
Verhältnis Enzym : Substrat (G/G) = 0,01 – 0,20 : 1 Reaktionstemperatur : Gefrierpunkt der wässrigen Phase bis ca. 40°, insbesondere ca. 0-10° über Gefrierpunkt
Aufarbeitung : Extraktion des Monoacylats mit organischem Lösungsmittel und Abdampfen des letzteren.

Das benötigte Enzym kann natürlich auch in immobilisierter Form eingesetzt werden.

Wie bereits erwähnt, sind die Verbindungen I wertvolle Zwischenprodukte. Sie lassen sich, aufgrund der Kohlenstoffzahl des Grundgerüstes, als optisch aktive $C_4$-Bausteine bezeichnen. Sie können zur Synthese verschiedener Naturstoffe oder synthetischer Arzneimittel, welche Methyl-gruppen-tragende chirale Kohlenstoffatome enthalten, verwendet werden (Q. Branca, A. Fischli, Helv. Chim. Acta 60, 925 (1977), D.A. Evans, C.E. Sachs, W.A. Kleschick, T.R. Taber, J. Am. Chem. Soc. 101, 6789 (1979), D.B. Collum. J.H. Mc Donald III, W.C. Still, J. Am. Chem. Soc. 102, 2117, 2118, 2120 (1980), C-Y. Byon, M. Gut, V. Toome. J. Org. Chem. 46, 3901 (1981), A.P. Kozikowski & Y.-Y. Chem. J. Org. Chem. 46, 5248 (1981), A.I. Meyers, J.P. Hudspeth, Tetrahedron Lett. 22 3925 (1981), H. Nagaoka, Y. Kishi, Tetrahedron 37, 3873 (1981), M. Kato, K. More, Agric. Biol. Chem. 49, 2479 (1983)). Ein wichtiger Vorteil der Verbindungen I besteht darin, dass sowohl das Kohlenstoffatom 1 wie das Kohlenstoffatom 3 die Oxidationsstufe eines Alkoholes aufweisen. Die bisher zur Verfügung stehenden optisch aktiven $C_4$-Bausteine waren hauptsächlich Derivate der β-Hydroxy-isobuttersäure (also eine Säure-Oxidationsstufe am Kohlenstoffatom 1), was bei der weiteren Verwendung gewöhnlich einen Reduktionsschritt mit einem (teuren) Hydridreagens erforderlich machte (vgl. A. Fischli, "Modern Synthetic Methods 1980", Vol. 2, Ed. R. Scheffold, Salle & Sauerländer, Frankfurt am Main & Aarau, 1980, S. 269 ff). Mit den neuen Verbindungen I erübrigt sich dieser Reduk- tionsschritt.

Die Verbindungen I können insbesondere bei der Synthese von naturidentischem Vitamin E, also (R,R,R)-α-Tocopherol, Verwendung finden. So lässt sich der "$C_{14}$"-Seitenkettenkettenalkohol, also die Verbindung (R,R)-2,6,10-Trimethyl-1-undecanol, z.B. leicht aus einer Verbindung I gemäss dem attraktiven Verknüpfungsschema $C_4 + C_{10} \rightarrow C_{14}$ aufbauen. Dazu wird die Hydroxylgruppe in I in geeigneter Weise aktiviert (z.B. als p-Toluolsulfonat) und die Acylgruppe durch Verseifung und anschliessendes Schützen der freigesetzten Hydroxylgruppe (z.B. als Tetrahydropyranyläther) durch eine stabile Schutzgruppe ersetzt. Ein derartig modifizierter $C_4$-Baustein wird dann, in bekannter Weise, mit einem $C_{10}$-Grignardreagens, also z.B. (R)-3,7-Dimethyloctylmagnesiumbromid, verknüpft, wobeinach Entfernung der Schutzgruppe der gewünschte "$C_{14}$"-Seitenkettenalkohol erhalten wird. Dies ist im unten stehenden Beispiel D exemplifiziert. Dieser "$C_{14}$"-Seitenkettenalkohol ist ein gesuchtes Zwischenprodukt für (R,R,R)-α-Tocopherol (N. Cohen et al., J. Org. Chem. 41, 3505 (1976). Die Verbindungen I lassen sich aber auch in vielfältiger Weise anders derivatisieren und modifizieren. Beispielsweise führt eine Umkehrung der eben erwähnten Manipulationen, also 1) Schützen der freien Hydroxylgruppe (z.B. als Tetrahydropyranyläther), 2) Verseifen der Acylgruppe und 3) Aktivieren der freigesetzten Hydroxylgruppe (z.B. als p-Toluolsulfonat) zu $C_4$-Bausteinen der enantiomeren Reihe.

Ein weiterer interessanter Weg führt über das neue Tritylätherderivat IV zum "$C_{14}$"-Seitenkettenalkohol

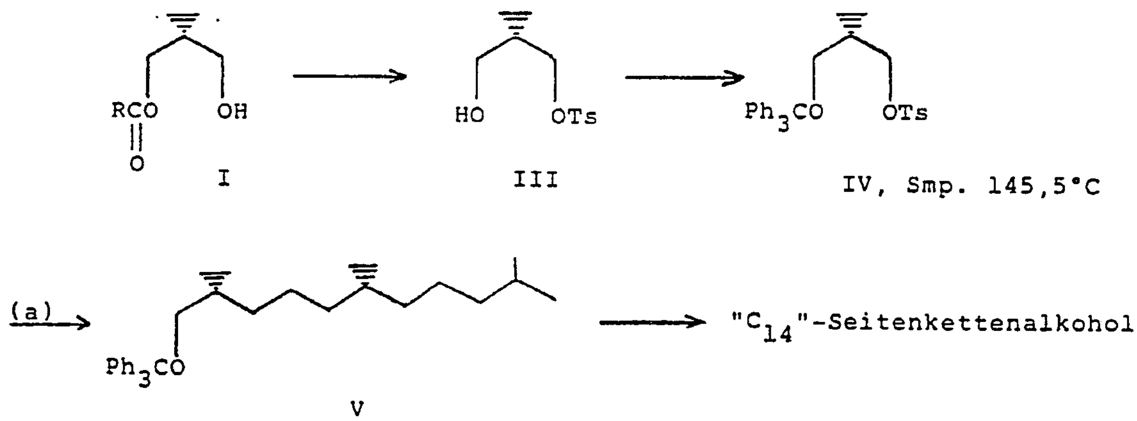


I — III — IV, Smp. 145,5°C

V

Ts = Tosyl
Ph = Phenyl
(a) = (R)-3,7-Dimethyl-octylmagnesiumbromid. Katalysator z.B. $Li_2CuCl_4$

Der Vorteil dieses Weges besteht in der Tatsache, dass das Produkt IV kristallin ist, durch Kristallisation auf > 99% enantiomere Reinheit anreicherbar ist, und das Verhältnis R/S von V so bis auf 99,5 : 0,5 erhöht werden kann. Ergebnisse beispielsweise erzielt mit R = n-Propyl, aber unabhängig von der Natur von R.

Weiterhin lassen sich $C_5$-Bausteine durch Austausch, z.B. der p-Toluolsulfonyloxygruppe durch die Cyanogruppe einfach herstellen.

Die Verbindungen I fallen aus dem prochiralen Substrat in einfacher Weise in hohen chemischen Ausbeuten, z.B. > 90% I an. Die Werte von ee sind im allgemeinen über 90%.

## Beispiele

### A. Herstellung der Diester II

#### a) 2-Methyl-1,3-propandiol

2-Methyl-1,3-propandiol wurde durch Hydrierung von Methylmalonsäure-diäthylester (H. Adkins & H.R. Billica, J. Am. Chem. Soc. 70, 3121 (1948)) hergestellt: Eine Lösung von 175 g (1.0 Mol) Methylmalonsäure-diäthylester in 1,3 l abs. Aethanol wurde im Autoklaven in Gegenwart von 260 g Kupferchromit (Girdlar, Typ 99B) während 9 Stunden bei 160°C und 280 Atm Druck hydriert. Nach Filtration vom Katalysator wurde eingedampft und der Rückstand wurde destilliert. Es wurden 70,35 g (78%) 2-Methyl-1,3-propandiol als farbloses Oel vom Siedepunkt 100-110°C/10 Torr erhalten; Reinheit: 97% (GC).

#### b) 2-Methyl-1,3-propandiol-dibutyrat (Buttersäure-(2-methyl-1,3-propandiol)ester)

Zu einer Lösung von 70 g (0,753 Mol) 2-Methyl-1,3-propandiol und 1,8 g (0,015 Mol) 4-Dimethylaminopyridin in 243 ml (238,2 g, 3,0 Mol) Pyridin wurden bei 0°C während 1 Stunde 262,2 g (1,656 Mol) Buttersäureanhydrid zugetropft. Das Gemisch wurde 16 Stunden bei Raumtemperatur gerührt und am Rotationsverdampfer eingedampft. Der Rückstand wurde in 500 ml Aether aufgenommen und die Lösung wurde mit gesättigter $NaHCO_3$-Lösung, 1N HCl und Wasser gewaschen, über $MgSO_4$ getrocknet, filtriert und

eingedampft. Eine Destillation am Hochvakuum lieferte 152,2 g (88%) 2-Methyl-1,3--propandiol-dibutyrat als farblose Flüssigkeit, Siedepunkt 78°C/0,8 Torr ; GC-Reinheit 99,5%.

c) In analoger Weise wurden die folgenden Diacylderivate von 2-Methyl-1,3-propandiol ausgehend von den entsprechenden Säureanhydriden hergestellt :

2-Methyl-1,3-propandiol-diacetat
Siedepunkt 102-104°C/8-9 mm ; Analyse ber. für $C_8H_{14}O_4$ (174,22) : C 55,16, H 8,10 ; gef. : C 55,43, H 8,30%.
2-Methyl-1,3-propandiol-dipropionat
72% Ausbeute ; Analyse ber. für $C_{10}H_{18}O_4$ (202,25) : C 59,39, H 8,97 ; gef. C 59,35, H 8,76%.
2-Methyl-1,3-propandiol-divalerat
66% Ausbeute ; Sdp. ca. 100°C/0,4 Torr.

B. Enzymatische Hydrolyse

Beispiel 1

70 g (304 mMol) 2-Methyl-1,3-propandiol-dibutyrat wurden in 8,4 l 0,1 M Guanidiniumrhodanid und 350 ml 0,1 M Natriumphosphatpuffer bei pH 7 emulgiert und die Emulsion auf 0°C abgekühlt. Der pH-Wert wurde durch Zugabe von 0,1 N NaOH auf 7,0 ajustiert. Der Start der enzymatischen Reaktion erfolgte durch Zugabe von 7,0 g Lipase P30 (Amano Pharmaceutical Co. Ltd.). Unter vigorosem Rühren des Reaktionsgemisches wurde der pH-Wert mittels Zudosieren von 1,0 N NaOH konstant bei 7,0 gehalten. Die Reaktion wurde nach einem Verbrauch von 304 ml 1N NaOH (= 50% Umsatz bez. Esteräquivalenten ; nach 1 Stunde) durch Zugabe von 3 l Dichlormethan abgebrochen. Es erfolgte Extraktion des Produkts mit 2 × 3 l Dichlormethan (Beschleunigung der Phasentrennung mittels kurzer Zentrifugation). Die organische Phase wurde über $MgSO_4$ getrocknet, eingeengt und der Rückstand im Hochvakuum destilliert (Badtemperatur : 90°C). Ausbeute : 45 g (92%) Buttersäure-[(R)-3-hydroxy-2-methylpropyl]ester ; Reinheit : 99,9% (GC). $[\alpha]^{20}_{365}$ = – 28,9° (1% in Aethanol). Enantiomerenreinheit = 95,7% ee (über Diastereomeren-Bestimmung) ; Analyse ber. für $C_8H_{16}O_3$ (160.21) : C 59,98, H 10,07 ; gef. : C 59,97, H 9,98%.

Beispiel 2

6,5 g (28 mMol 2-Methyl-1,3-propandiol-dibutyrat wurden in 775 ml 75 mM LiSCN und 25 ml 0,1 M Natriumphosphatpuffer bei pH 8 emulgiert und die Emulsion auf 0°C abgekühlt. Die Hydrolyse wurde durch Zugabe von 500 mg Lipase P30 eingeleitet. Das Reaktionsgemisch wurde vigoros bei 0°C gerührt und der pH-Wert durch Zudosieren von 1,0 N NaOH (pH-Stat) bei 8,0 gehalten. Nach einem Verbrauch von 28,3 ml 1N NaOH (50% Umsatz bez. Esteräquivalenten ; nach ca. 70 Minuten) wurde die Reaktion durch Zugabe von 300 ml Dichlormethan abgebrochen und das Produkt mit 2 × 300 ml Dichlormethan extrahiert. Die organischen Phasen wurden vereinigt, über $MgSO_4$ getrocknet, eingeengt und der Rückstand im Kugelrohr destilliert. Ausbeute : 4,2 g (93%) Buttersäure-[(R)-3-hydroxy-2-methylpropyl]ester. $[\alpha]^{20}_{365}$ = – 27,9° (1% in Aethanol) ; Enantiomerenreinheit : 94% ee.

Beispiele 3-6

200 mg (688 mMol) 2-Methyl-1,3-propandiol-dibutyrat wurden in 25 ml A (Beisp. 3), B (Beisp. 4), C (Beisp. 5) oder D (Beisp. 6) und 1 ml 0,1 M Natriumphosphatpuffer pH 7 (Beisp. 3, 5) oder pH 8 (Beisp. 4, 6) emulgiert und die Emulsion auf 4°C (Beisp. 6), 0°C (Beisp. 3, 5) oder –2° bis –3°C (Beisp. 4) gekühlt. Die Hydrolyse wurde durch Zugabe von 6,1 mg (Beisp. 6) oder 30,5 mg (Beisp. 3-5) Lipase P30 eingeleitet. Das Reaktionsgemisch wurde bei der gewählten Temperatur vigoros gerührt und der pH-Wert durch Zudosieren von 0,1 N NaOH konstant gehalten. Nach einem Verbrauch von 8,68 ml 0,1 M NaOH (50% Umsatz bez. Esteräquivalenten) wurde die Reaktion durch Zugabe von 25 ml Dichlormethan abgebrochen und das Produkt mit 2 × 25 ml Dichlormethan extrahiert. Die organischen Phasen wurden vereinigt, über $MgSO_4$ getrocknet und am Rollverdampfer eingedampft. Die Ausbeuten an Buttersäure-[(R)-3-hydroxy-2-methylpropyl]ester betrugen 130-135 mg (93,5-97%).

| Beispiel | Medium (Zusammensetzung) | pH | Temperatur °C | $[alpha]_{365}^{20}$ (1% in Aethanol) | % ee |
|---|---|---|---|---|---|
| 3 | A : Guanidiniumchlorid 0,1M | 7,0 | 0° | -28,0° | 96,5 |
| 4 | B : LiSCN 0,2M; AetOH 0,1M | 8,0 | -2 bis -3° | -29,0° | 96,3 |
| 5 | C : Natriumcitrat pH 7; 0,1M | 7,0 | 0° | -27,8° | 95,6 |
| 6 | D : Glycerin 0,1M 8,0 | 8,0 | 4° | | 95,2 |

C. Konfigurationskorrelation ((S)-3-Hydroxy-2-methylpropyl)-phenylcarbamat

Eine Lösung von 0,80 g (5,0 mMol) (–)-Buttersäure-[(R)-3-hydroxy-2-methylpropyl) ester ($[alpha]_{365}^{20}$ = –27,9° [1% in Aethanol]) und 0,60 g (5,0 mMol) frisch destilliertem Phenylisocyanat in 10 ml trockenem Benzol wurde 20 Stunden bei Raumtemperatur gerührt. Nach Eindampfen zur Trockene wurde der Rückstand in Hexan/Aether 1 : 1 aufgenommen, das Gemisch filtriert, das Filtrat eingedampft und der Rückstand mit Hexan/Aether 1 : 1 an Kieselgel chromatographiert. Es wurden 1,20 g (86%) ((S)-3-Butyryloxy-2-methylpropyl)-phenylcarbamat als farbloses Oel erhalten.

Eine Lösung von 0,58 g (2,0 mMol) dieses Materials in 2 ml Methanol wurde mit 2 ml einer 1N methanolischen KOH-Lösung versetzt. Nach 15 Minuten wurde mit 0,12 ml Essigsäure versetzt, filtriert und eingedampft. Der Rückstand aus dem Filtrat wurde mit Dichlormethan/Aether 95 : 5 und mit Aether an Kieselgel chromatographiert. Dabei wurden nach Trocknung am Hochvakuum 0,38 g (91%) ((S)-3-Hydroxy-2-methylpropyl)-phenylcarbamat als farbloses Oel erhalten ; $[\alpha]_D^{20}$ = –4,66° (2,44% in Benzol) ; $[\alpha]_D^{20}$ = +3,98° (2,03% in Methanol). Für das enantiomere ((R)-3-Hydroxy-2-methylpropyl)-phenylcarbamat werden Drehwerte von $[\alpha]_D^{25}$ = 6,0° (2,0% in Benzol) und $[\alpha]_D^{25}$ = –4,22° (2,0% in Methanol) berichtet (N. Cohen et al., J. Org. Chem. 41, 3505 (1976)]. Somit ist die Absolutkonfiguration des (–)-Buttersäure-[(R)-3-hydroxy-2-methylpropyl)-esters bewiesen ; optische Reinheit ca. 78-94%.

D. Synthese von (R,R)-2,6,10-Trimethyl-1-undecanol

a) Buttersäure-[(S)-2-methyl-3-(p-toluolsulfonyloxy)-propyl]ester

Zu einer Lösung von 2,0 g (12,18 mMol) Buttersäure-[(R)-3-hydroxy-2-methylpropyl)ester ($[\alpha]_{365}^{20}$ = –27,1° (1% in Aethanol)) und 1,97 ml (24,36 mMol) Pyridin in 15 ml Chloroform wurden bei 0° 3,5 g (18,27 mMol) p-Toluolsulfochlorid in kleinen Portionen zugegeben und das Reaktionsgemisch wurde 3 Stunden bei 0° gerührt. Anschliessend wurde mit 50 ml Aether und mit 10 ml Wasser versetzt, die Phasen getrennt und die organische Phase wurde mit 1,5N HCl, gesättigter $NaHCO_3$-Lösung und Wasser gewaschen, über $MgSO_4$ getrocknet, filtriert und eingedampft. Der Rückstand wurde mit Hexan/Aether 7 : 3 an Kieselgel chromatographiert. Nach Trocknung am Hochvakuum wurden 3,20 g (83,5%) Buttersäure-[(S)-2-methyl-3-(p-toluolsulfonyloxy)-propyl]ester als farbloses Oel erhalten ; $[\alpha]_D^{20}$ = +5,2° (1% in Aethanol). Analyse ber. für $C_{15}H_{22}O_5S$ (314, 396) : C 57,31, H 7,05, S 10,20 ; gef. C 57,33, H 7,15, S 10,12%.

b) p-Toluolsulfonsäure-[(S)-3-hydroxy-2-methylpropyl]ester

Eine Lösung von 2,70 g (8,59 mMol) Buttersäure-[(S)-2-methyl-3-(p-toluolsulfonyloxy)-propyl]ester in 10 ml Methanol wurde bei 0°C mit 4,8 ml einer 1,8 N methanolischen KOH-Lösung versetzt und 45 Minuten bei 0°C gerührt. Anschliessend wurde mit 0,55 ml Essigsäure versetzt, am Rotationsverdampfer zur Trockene eingeengt, der Rückstand in Aether aufgenommen, vom ausgefallenen Festkörper filtriert und das Filtrat eingedampft. Der Rückstand (2,1 g) wurde mit Hexan/Aether 3 : 7 an Kieselgel chromatographiert, wobei nach Trocknung am Hochvakuum bei 60° 1,90 g (90,5%) p-Toluolsulfonsäure-[(S)-3-hydroxy-2-methylpropyl]ester als farbloses Oel anfielen ; $[\alpha]_D^{20}$ = +12,1° (1% in Aethanol). [Für den enantiomeren p-Toluolsulfonsäure-[(R)-3-hydroxy-2-methylpropyl]ester wird ein Drehwert $[\alpha]_D^{RT} = -9,7°$ (2.93% in Aethanol) berichtet ; vgl. C. Najera, M. Yus & D. Seebach, Helv. Chim. Acta 67, 289 (1984)]. Analyse ber. für $C_{11}H_{16}O_4S$ (224, 305) ; C 54,08, H 6,60, S 13,12 ; gef. : C 53,65, H 6,72, S 13,04%.

c) p-Toluolsulfonsäure-{(S)-2-methyl-3-[(tetrahydro-2H-pyran-2-yl)oxy]propyl}ester

Eine Lösung von 1,70 g (6,96 mMol) p-Toluolsulfonsäure-[(S)-3-hydroxy-2-methylpropyl]ester, 0,878 g (10,4 mMol) Dihydropyran und 175 mg Pyridinium-(p-toluolsulfonat) in 50 ml Dichlormethan wurde 5 Stunden bei Raumtemperatur gerührt. Dann wurde mit 100 ml Aether und 50 ml halbgesättigter NaCl-Lösung versetzt, die Phasen getrennt und die organische Phase wurde mit gesättigter NaCl-Lösung gewaschen, über $MgSO_4$ getrocknet, filtriert und eingedampft. Chromatographie des Rückstandes an Kieselgel mit Hexan/Aether 3 : 7 lieferte nach Trocknung am Hochvakuum 1,90 g (83,3%) p-Toluolsulfonsäure-{(S)-2-methyl-3-[(tetrahydro-2H-pyran-2-yl)oxy]propyl}ester als farbloses, viskoses Oel, welches beim Stehen bei 0°C kristallisierte ; $[\alpha]_D^{20} = +5,7°$ (1% in $CHCl_3$). Analyse ber. für $C_{16}H_{24}O_5S$ (328. 423) : C 58,51, H 7,37, S 9,76 ; gef. : C 58,64, H 7,45, S 9,67%.

d) (R,R)-2,6,10-Trimethyl-1-undecanol

Eine Suspension von 234 mg (9,62 mMol) Magnesiumspänen in 1,5 ml trockenem Tetrahydrofuran wurde unter Argonatmosphäre mit 20 µl 1,2-Dibromäthan versetzt und 15 Minuten gerührt. Nach Entfernung der überstehenden Lösung wurden die Magnesiumspäne zweimal mit je 1,5 ml Tetrahydrofuran gewaschen und erneut in 0,75 ml Tetrahydrofuran aufgeschlämmt. Anschliessend wurde eine Lösung von 1,50 g (6,78 mMol) (R)-1-Brom-3,7-dimethyloctan (von 97% optischer Reinheit) in 7,8 ml Tetrahydrofuran so zugetropft, dass die Reaktionstemperatur 30°C nicht überstieg. Nach einer Dauer von 2 Stunden bei Raumtemperatur wurde die so bereitete Grignardlösung mittels einer Kanüle zu einer auf −78°C gekühlten Lösung von 1,50 g (4,57 mMol) p-Toluolsulfonsäure-{(S)-2-methyl-3-[(tetrahydro-2H-pyran-2-yl)oxy]propyl}ester in 6,25 ml Tetrahydrofuran transferiert. Dann wurde 1,4 ml einer 0,1 M Lösung von Dilithiumtetrachlorocuprat zugefügt. Man liess unter Rühren innerhalb von 2 Stunden auf Raumtemperatur erwärmen, rührte noch 2 Stunden bei Raumtemperatur und versetzte dann mit 16 ml gesättigter $NH_4Cl$-Lösung und 100 ml Aether. Die Phasen wurden getrennt und die organische Phase wurde über $MgSO_4$ getrocknet, filtriert und eingedampft. Chromatographie des Rückstandes an Kieselgel mit Hexan/Aether 7 : 3 lieferte 1,1 g 2-[(R,R)-2,6,10-Trimethylundecyloxy]-tetrahydro-2H-pyran sowie aus den polareren Fraktionen 0,50 g (33%) nicht-umgesetzter p-Toluolsulfonsäure-{(S)-2-methyl-3-[(tetrahydro-2H-pyran-2-yl)oxy]propyl}ester.

Das erhaltene 2-[(R,R)-2,6,10-Trimethylundecyloxy]tetrahydro-2H-pyran (1,1 g) wurde in 6 ml Aethanol gelöst, mit 100 mg Pyridinium-(p-toluolsulfonat) versetzt, und die Lösung wurde 2 Stunden auf 60°C erhitzt. Anschliessend wurde eingedampft, in Aether aufgenommen, mit halbgesättigter NaCl-Lösung gewaschen, über $MgSO_4$ getrocknet, filtriert und eingedampft. Chromatographie an Kieselgel mit Hexan/ Aether 7 : 3 gefolgt von Destillation im Kugelrohr bei ca. 135°/0,2 Torr lieferte 0,42 g (R,R)-2,6,10-Trimethyl-1-undecanol als farblose Flüsigkeit ; Ausbeute 64,5% bezüglich umgesetztem p-Toluolsulfonsäure-{(S)-2-methyl-3-[(tetrahydro-2H-pyran-2-yl)oxy]propyl}ester. $[\alpha]_D^{20} = +9,12°$ (2,3% in Hexan) ; [Literaturwert : $[\alpha]_D^{25} = +9,13°$ (2,179 in Hexan); vgl. N. Cohen et al., J. Org. Chem., 41, 3505 (1976)]. Die diastereomere Reinheit wurde durch GC-Analyse zu 94,5% bestimmt, woraus sich 2R/2S- und 6R/6S-Verhältnisse von 97 : 3 und 98,5 : 1,5 errechnen lassen. Analyse ber. für $C_{14}H_{30}O$ (214, 393) : C 78,43, H 14,10 ; gef. C 78,40, H 14,28%.

Beispiel 7

175,6 mg (868 µMol) Dipropionat II wurden in 25 ml 0,1 M NaCl und 1 ml 0,1 M Natriumphosphatpuffer pH 7 emulgiert und die Hydrolyse durch Zugabe von 6,1 mg Lipase P30 gestartet. Das Reaktionsgemisch wurde kräftig gerührt und der pH-Wert durch Zudosieren von 0,1 N NaOH konstant gehalten. Nach einem Verbrauch von 8,36 ml 0,1 N NaOH (48% Umsatz bez. Esteräquivalenten) wurde die Reaktion abgebrochen und das Produkt wie oben aufgearbeitet. Die spez. Drehung des erhaltenen Oels war $[\alpha]_{365}^{25} = -25,7°$ (1,0% in Aethanol) bei einer Reinheit von 96,3% (GC). Die Enantiomerenreinheit (Diastereomerenbestimmung) des Propionats I betrug 86,1% ee.

Beispiel 8

224,1 mg (868 µMol) Divalerat II wurden gemäss Beispiel 7, jedoch bei 0°C und mit 30,7 mg Lipase P30,

umgesetzt (Abbruch der Reaktion nach Zugabe von 8,68 ml 0,1 N NaOH = 50% Umsatz bez. Esteräquivalenten). Die spezifische Drehung des erhaltenen Oels betrug $[\alpha]_{365}^{25} = -24{,}1°$ (1,0% in Aethanol) bei einer Reinheit von 99% (GC). Die Enantiomerenreinheit (Diastereomerenbestimmung) des Valerats I betrug 91,7% ee.

## Patentansprüche

1. Verbindungen der Formel

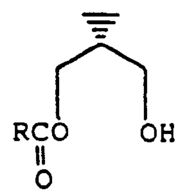

I

worin R $C_{1-10}$-Alkyl, insbesondere $C_{1-5}$-Alkyl, darstellt.

2. Essigsäure-[(R)-3-hydroxy-2-methylpropyl]ester.

3. Propionsäure-[(R)-3-hydroxy-2-methylpropyl]ester.

4. Buttersäure-[(R)-3-hydroxy-2-methylpropyl]ester.

5. Valeriansäure-[(R)-3-hydroxy-2-methylpropyl]ester.

6. Verfahren zur Herstellung der Verbindungen

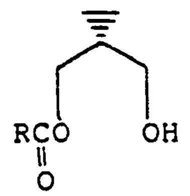

I

worin R $C_{1-10}$-Alkyl, insbesondere $C_{1-5}$-Alkyl, darstellt, dadurch gekennzeichnet, dass man einen symmetrischen Diester der Formel

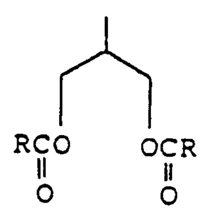

II

worin R obige Bedeutung besitzt, mit einer Lipase, vorzugsweise einer mikrobiellen Lipase des Genus Pseudomonas, behandelt.

7. Verfahren nach Anspruch 6, dadurch gekennzeichnet, dass man mit einer Lipase aus Pseudomonas fluorescens arbeitet.

8. Verfahren nach Anspruch 5, dadurch gekennzeichnet, dass man mit einer Lipase des Genus Rhizopus oder des Genus Mucor arbeitet.

9. Verfahren nach Anspruch 6-8, dadurch gekennzeichnet, dass R Methyl darstellt.

10. Verfahren nach Anspruch 6-8, dadurch gekennzeichnet, dass R Aethyl darstellt.

11. Verfahren nach Anspruch 6-8, dadurch gekennzeichnet, dass R n-Propyl darstellt.

12. Verfahren nach Anspruch 6-8, dadurch gekennzeichnet, dass R Butyl darstellt.

13. Verfahren zur Herstellung von (R,R,R)-α-Tocopherol, dadurch gekennzeichnet, dass man eine Verbindung der Formel

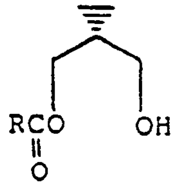

I

worin R $C_{1-10}$-Alkyl, insbesondere $C_{1-5}$-Alkyl, darstellt, nach dem Verknüpfungsschema $C_4 + C_{10}$ in den $C_{14}$-Seitenkettenalkohol (R,R)-2,6,10-Trimethyl-1-undecanol überführt und letzteren in an sich bekannter Weise in (R,R,R)-α-Tocopherol überführt.

## Claims

1. Compounds of the formula

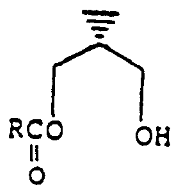

I

wherein R represents $C_{1-10}$-alkyl, especially $C_{1-5}$-alkyl.

2. (R)-3-Hydroxy-2-methylpropyl acetate.
3. (R)-3-Hydroxy-2-methylpropyl propionate.
4. (R)-3-Hydroxy-2-methylpropyl butyrate.
5. (R)-3-Hydroxy-2-methylpropyl valerate.
6. A process for the manufacture of the compounds

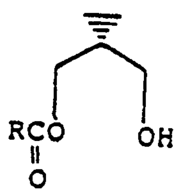

I

wherein R represents $C_{1-10}$-alkyl, especially $C_{1-5}$-alkyl, characterized by treating a symmetrical diester of the formula

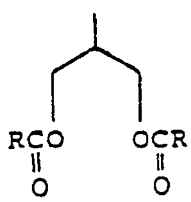

II

wherein R has the above significance, with a lipase, preferably a microbial lipase of the genus Pseudomonas.

7. A process according to claim 6, characterized in that a lipase from Pseudomonas fluorescens is used.
8. A process according to claim 5, characterized in that a lipase of the genus Rhizopus or of the genus Mucor is used.
9. A process according to claim 6-8, characterized in that R represents methyl.
10. A process according to claim 6-8, characterized in that R represents ethyl.

11. A process according to claim 6-8, characterized in that R represents n-propyl.

12. A process according to claim 6-8, characterized in that R represents butyl.

13. A process for the manufacture of (R,R,R)-α-tocopherol, characterized by converting a compound of the formula

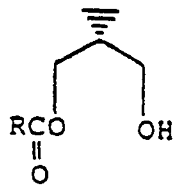

I

wherein R represents $C_{1-10}$-alkyl, especially $C_{1-5}$-alkyl, according to the linkage scheme $C_4 + C_{10}$ into the $C_{14}$ side-chain alcohol (R,R)-2,6,10-trimethyl-1-undecanol and converting the latter into (R.R,R)-α-tocopherol in a manner known per se.

**Revendications**

1. Composés de formule

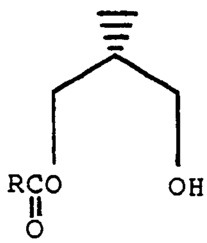

I

dans laquelle R représente un groupe alkyle en $C_1$-$C_{10}$, plus spécialement en $C_1$-$C_5$.

2. L'acétate de (R)-3-hydroxy-2-méthylpropyle.

3. Le propionate de (R)-3-hydroxy-2-méthylpropyle.

4. Le butyrate de (R)-3-hydroxy-2-méthylpropyle.

5. Le valérate de (R)-3-hydroxy-2-méthylpropyle.

6. Procédé de préparation des composés de formule

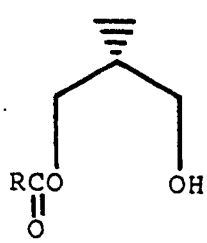

I

dans laquelle R représente un groupe alkyle en $C_1$-$C_{10}$, plus spécialement en $C_1$-$C_5$, caractérisé en ce que l'on traite un diester symétrique de formule

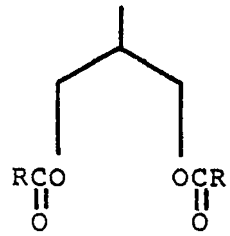

II

dans laquelle R a les significations indiquées ci-dessus, par une lipase, de préférence une lipase microbienne du genre Pseudomonas.

7. Procédé selon la revendication 6, caractérisé en ce que l'on opère avec une lipase de Pseudomonas fluorescens.

8. Procédé selon la revendication 5, caractérisé en ce que l'on opère avec une lipase du genre Rhizopus ou du genre Mucor.

9. Procédé selon les revendications 6 à 8, caractérisé en ce que R représente un groupe méthyle.

10. Procédé selon les revendications 6 à 8, caractérisé en ce que R représente un groupe éthyle.

11. Procédé selon les revendications 6 à 8, caractérisé en ce que R représente un groupe n-propyle.

12. Procédé selon les revendications 6 à 8, caractérisé en ce que R représente un groupe butyle.

13. Procédé de préparation du (R,R,R)-alpha-tocophérol, caractérisé en ce que l'on convertit un composé de formule

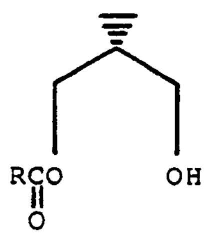

I

dans laquelle R représente un groupe alkyle en $C_1$-$C_{10}$, plus spécialement en $C_1$-$C_5$,
selon le schéma de liaison $C_4$ + $C_{10}$, en l'alcool en $C_{14}$ à chaîne latérale (R.R)-2,6,10-triméthyl-1-undécanol, qu'on convertit ensuite de manière connue en soi en le (R,R,R)-alpha-tocophérol.